# EUROPEAN PATENT SPECIFICATION

(11) **EP 2 056 923 B1**
(45) Date of publication and mention of the grant of the patent: **11.12.2019**
(21) Application number: 07803053.3
(22) Date of filing: 30.08.2007
(51) Int. Cl.: A61M 39/10

(54) **ARTERIOVENOUS FISTULA**
ARTERIOVENÖSE FISTEL
FISTULE ARTÉRIOVEINEUSE

(30) Priority: 30.08.2006 GB 0617074
(43) Date of publication of application: 13.05.2009
(73) Proprietor: Angiomed GmbH & Co. Medizintechnik KG, 76227 Karlsruhe (DE)
(72) Inventor: DORN, Jürgen, 68809 Neulussheim (DE)
(74) Representative: Hoffmann Eitle
(86) International application number: PCT/EP2007/059044
(87) International publication number: WO 2008/025818

(56) References cited:
- WO-A-02/02163
- WO-A-96/28116
- WO-A-2005/000165
- WO-A-2005/011788
- JP-A- 2003 245 343
- US-A- 6 036 721
- US-A1- 2004 199 177
- US-B1- 6 190 353

## Description

### Technical Field

This invention relates to a shunt for providing an arteriovenous fistula, a system for placing the shunt and a method for placing the shunt. The features of the pre-characterizing portion of claim 1 are found in document JP 2003-245343.

Individuals suffering from renal failure undergo haemodialysis, in which a dialysis machine performs the function of the failed kidneys. Haemodialysis has to be repeated periodically and frequently and this requires repeated puncturing of the arteriovenous system using relatively large diameter dialysis needles. Frequent puncturing of native vessels with large bore needles can cause trauma and eventually a loss of patency of those vessels.

### Background Art

It has previously been proposed to provide, externally of the body, an arteriovenous shunt that can be accessed with a dialysis needle, thereby to relieve the individual of the adverse effects of repeated puncturing of native vessels. However, there remains the problem of reliable sealing of the synthetic shunt wall, and the risks of infection arising from the percutaneous nature of the synthetic shunt. It is an object of the present invention to mitigate these disadvantages.

JP 2003-245343 aims to create a subcutaneous shunt structure for dialysis, using a self-expanding stent, bare at its ends and covered in a mid-length portion that shunts between a vein and an artery in the forearm of a patient. The shunt is to be placed using a hollow hypodermic needle which is advanced through the vein and into the artery. A guidewire is to be advanced through a bore of the needle and beyond. The needle is to be withdrawn, leaving the guidewire in place. A balloon device is then to be advanced over the wire and, when its leading end is in the artery, it is to be inflated to make room for the shunt, then deflated and retracted. Then, a covered stent, catheter-based, delivery system is to be advanced over the guidewire till the stent shunt is in position, then an outer sheath around the stent is withdrawn, to release the self-expanding stent, thereby installing the shunt. The catheter and the guidewire are then to be removed.

WO 02/02163 discloses creating a fistula between a vein and an artery using a catheter in the vein and another in the artery. One of the catheters delivers a stent to the site of the fistula and places it in the vein. The procedure is for arterializing a vein, in treatment of peripheral vascular disease, as a means of avoiding amputation of the diseased limb, for example, the foot.

WO 2007/087368 has a similar disclosure, with magnets to bring together the venous and arterial catheters together.

WO 2005/000165 discloses a stent covered with inner and outer layers of PTFE with a marker sandwiched between the two layers .

WO 2005/044076 discloses an expandable prosthesis equipped with a visual maker to be viewed by an endoscope.

US-A-6264684 discloses self-expanding stent grafts. The graft material can be expanded PTFE and the stent material can be a shape memory alloy.

WO 98/34676 discloses a subcutaneous arteriovenous fistula with a needle-receiving access site to be punctured by a needle for haemodialysis, within the context of the known Squitieri haemodialysis and vascular access system.

WO 96/28116 A1 describes an intraluminal prosthesis comprising a tubular member having at least one end which is an oblique angle to the lumen. The prosthesis is inserted into a body lumen, such as a blood vessel, while compressed. The prosthesis is expanded within the lumen, and the oblique end of the prosthesis contacts the lumen at an oblique angle. This oblique angle reduces the stenotic effect of hyperplasia which generally occurs at the ends of intraluminal prostheses.

### Summary of the Invention

The present invention is defined by the independent claim below being directed to a system for facilitating dialysis by creating an arteriovenous fistula with a shunt, the shunt having a dialysis needle landing zone. The dependent claims are directed to optional features and preferred embodiments.

In a first aspect, this invention provides a covered radially expansible stent, capable of being advanced along a bodily lumen, and capable of performing as an arteriovenous shunt. The shunt is one that is "stickable", that is to say, it can endure repeated puncturing with a dialysis needle. The shunt is located subcutaneously, so that the only element that is percutaneous during dialysis is the dialysis needle. One way to enhance sealing of the wall of the shunt, when dialysis is completed and the needle is withdrawn, is to provide a special gel between the puncture site and the skin overlaying it. The special gel may be the one marketed under the trademark EGRESS. The shunt resembles a stent graft, known per se, in which a metal stent matrix of struts is covered by a thin membrane of expanded polytetrafluoroethylene (ePTFE).

After placement, one end of the shunt lies inside an artery, and the other end of the shunt lies inside a vein. It will be appreciated that there is a blood pressure differential across the ends of the shunt and that the shunt end in the artery is at the high pressure end of the shunt. The known shunt has the shape of a loop that bends through about 180°, so that one of its open ends faces upstream in the artery and the other of its open ends faces downstream in the vein. However, with the present invention, it is contemplated that the shunt, after placement, will take a more or less straight line in its intermediate section bridging between the vein and the artery, its one open end facing downstream in the artery and its other open end also facing downstream, but in the vein.

Preferably, the cover on the stent does not extend all the way to the ends of the stent matrix. The uncovered end section of the stent matrix, at each end of the stent, serves as an anchor for the shunt, within the lumen of the respective artery and vein. Since the shunt should extend across from the artery to the vein, along a line that takes an acute angle with respect to the upstream length direction of the artery, the covering of the stent should stop short of the end of the stent inside the artery, at a line that is slanting with respect to the long axis of the stent, with the objective that the portion of the shunt that lies within the arterial lumen is an uncovered portion (whereby blood can flow through the open interstices of the stent mesh anchoring the shunt inside the lumen of the artery). At the downstream end of the shunt, where the stent is in the lumen of the vein, it is less important that the end section of the stent matrix is uncovered but, if it is, it is preferred that the end of the covering should be more or less aligned with the wall surface of the arterial lumen.

For placement of the shunt (as described below) it will be advantageous to equip the stent with at least one radiopaque marker and there may well be benefit in providing the stent with at least one visual marker, to be viewed directly by the unaided human eye, also as explained below. The visual marker will conveniently be of a strong colour, that stands out relative to adjacent areas of the stent. Since a covering of ePTFE is usually of a white colour, there is full scope for a range of coloured markers to indicate different parts of the stent.

Importantly, the system of the present invention contemplates delivering the arteriovenous shunt trans-luminally. It is characterised by a catheter for advancing a covered stent carried near the distal end of the catheter along a bodily lumen into a position in which the stent can function as an arteriovenous shunt between a first and a second bodily lumen, the catheter including means to puncture (that is to say, to broach) radially outwardly a wall of said first lumen and radially inwardly a wall of said second lumen, and then deploy the stent.

The way in which the arterial wall and the venous wall is broached can be by means of a penetrating point provided at the distal end of the catheter and, normally, the catheter includes means by which an operator at the proximal end of the catheter can move the point axially, between a distally advanced unsheathed penetrative disposition for penetrating first the wall of the artery and then the wall of the vein, and a proximally retracted sheathed non-penetrative disposition, in which the point will be located during the period when the catheter is advanced to the point where it is to broach the vascular wall.

Since the catheter will lie along the axis of the bodily lumen, yet is required to broach the wall of the lumen in which the catheter advances, the penetrative point should be capable of facing at an angle to the longitudinal axis of the distal end zone of the catheter, for breaching the wall where required. One convenient way of achieving this angle is to provide the penetrative point on the distal end of a needle of shape memory alloy such as nickel titanium, commonly used for building self-expanding stents. As the needle is advanced from the sheathed to the unsheathed disposition, it can revert to its remembered disposition, somewhat curved, thereby putting the penetrative tip at an angle to the long axis of the catheter.

Conveniently, the catheter will carry an obturator near its distal end, the purpose of the obturator being to expand the hole in the luminal wall made by the broaching means, in preparation for advance through the broached wall of the distal tip of the catheter proper, carrying the shunt. The obturator will likely have an olive shape, more or less atraumatic.

It will be appreciated that the shunt can be deployed analogously to a stent graft, that is to say, by proximal withdrawal of a confining sheath, once the covered stent has been placed by the catheter with its leading end within the venous lumen, its proximal end within the arterial lumen along which the catheter has been advanced, and with its intermediate length portion providing the fistula between the two lumens, and the needle stick zone for subsequently receiving the dialysis needle. After proximal withdrawal of the sheath, the covered stent expands radially and the delivery catheter, including obturator and penetrative point, can be proximally withdrawn through the lumen of the shunt.

The method of creating an arteriovascular fistula, with a shunt, in accordance with the present invention, is characterised by the steps of i) advancing a covered stent along a first lumen of the arteriovenous system ii) tunnelling through the wall of said lumen, through intervening tissue, and through the wall of a second lumen of the arteriovenous system to create the fistula, and iii) deploying the stent as a shunt in the fistula.

To enable the advance of the catheter containing the shunt, from the artery across to the vein, it is preferred to use a tunnelling device to tunnel between a percutaneous slit adjacent the artery where its wall is to be broached, and a second percutaneous slit adjacent the vein where the lumen wall of the vein is to be broached radially inwardly. With access provided with the two percutaneous slits, a tunnelling needle can be advanced form one slit to the other and it may be convenient to use the tunnelling needle not only to establish a channel through the tissue to receive the shunt delivery catheter, but also to install temporarily in that channel a sheath through which the delivery catheter can advance, said sheath being withdrawn afterwards, through one of the percutaneous slits.

After installation of the shunt, the various delivery components can all be removed, and the percutaneous slits closed and allowed to heal, so that the individual is left with a subcutaneous prosthesis which is an arteriovenous shunt, and the only percutaneous intervention needed is the temporary passage of the dialysis needle during dialysis procedures as such. After each dialysis treatment, the needle is withdrawn and the puncture site in the skin has the chance to heal.

The puncture site in the shunt does not have the capacity for natural healing but does have a capability for a high degree of sealing, through its natural resilience. The blood tightness of the punctured shunt can be enhanced by the provision of a pillow or pad of gel located between the puncture site of the shunt and the overlying cutaneous layers of natural tissue.

In this way, the present invention offers possibilities to enhance the quality of life of dialysis patients, and reduce the likelihood of percutaneous infection. Furthermore, by sparing arterial and venous wall tissue the trauma of repeated needle sticks, the likelihood of local stenosis of the arterial or venous lumens can be reduced.

For a better understanding of the present invention, and to show more clearly how the same may be carried into effect, reference will now be made, by way of example, to the accompanying drawings.

### Brief Description of the Drawings

- Fig. 1: is a sketch of an arteriovenous shunt,
- Fig. 2: is a sketch of an arteriovenous fistula,
- Fig. 3: is a sketch of an arteriovenous graft,
- Fig. 4: is a schematic diagram of a hemodialysis circuit,
- Fig. 5: is a diagram of a first embodiment of shunt according to the present invention,
- Fig. 6: is a photograph of the venous end of the shunt of Fig. 5 and depicts venous stent graft anastomosis,
- Fig. 7: is a photograph of the arterial end of the shunt of Fig. 5 and depicts arterial stent graft anastomosis,
- Fig. 8: shows the distal end of a delivery catheter for the shunt of Fig. 5, within an artificial lumen,
- Fig. 9: is a photograph of the Fig. 8 catheter distal end, but with a penetrating point in a penetrating disposition; and
- Fig. 10: is a photograph form the side, of the arterial end of the shunt shown diagrammatically in Fig. 5.

### Detailed Description

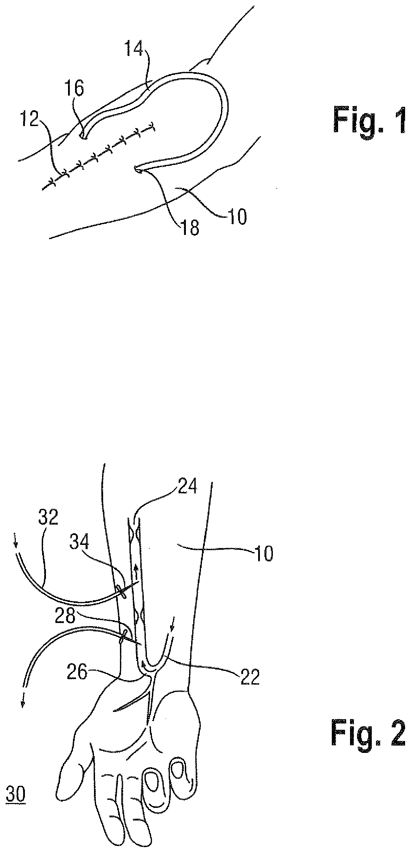

Looking first at Fig. 1, there is shown a human forearm 10 with a stitched together long percutaneous slit 12 and an arteriovenous shunt 14 with one end 16 extending through a percutaneous puncture into an arterial lumen of the forearm, and an opposite end 18 extending through a percutaneous puncture spaced from puncture 16, and into a venous lumen of the forearm. The shunt intermediate length portion is external of the body and therefore available for access by dialysis needles. However, the puncture sites 16 and 18 render the arrangement less than ideal from an infection perspective.

Turning to Fig. 2, there is shown again the human forearm 10 and, diagrammatically, beneath the skin, an arterial lumen 22, a venous lumen 24 and, between them, an arteriovenous fistula 26. A first dialysis needle 28 taps blood for a dialysis machine 30 and a return flow channel 32 from the machine delivers blood back to the patient via a second dialysis needle 34. In other words, blood flows from the patient via the first dialysis needle 28 to the dialysis machine and from the dialysis machine via the second dialysis needle 28 back to the patient.

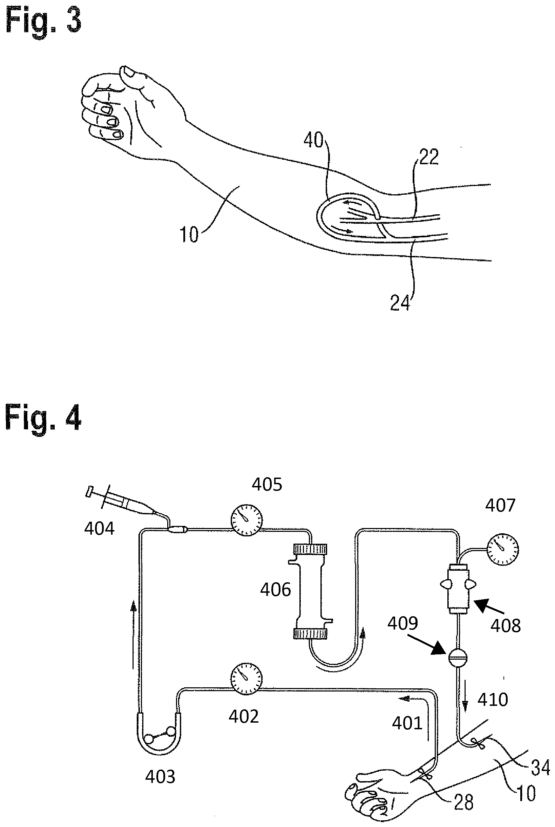

Looking at Fig. 3, we see the same human forearm 10, arterial lumen (artery) 22 and venous lumen (lumen) 24 but, this time, the
arteriovenous shunt is provided by a looped graft 40 which is located subcutaneously, and placed by open surgery.

Fig. 4 is included for the sake of completeness. It can be seen as the counterpart to what is shown in Fig. 2, with the same human forearm 10 being represented in the lower right hand corner of the diagram. Needle 28 withdraws blood from the machine, and needle 34 returns it to the body. Various components of the dialysis machine are known to those skilled in the art of access systems for dialysis and, in any case, are specified by reference signs 401 to 410 on the drawing Figure. Specifically, reference sign 401 denotes blood removed for cleansing, reference sign 402 denotes an arterial pressure monitor, reference sign 403 denotes a blood pump, reference sign 404 denotes a heparin pump (to prevent clotting), reference sign 405 denotes a dialyzer inflow pressure monitor, reference sign 406 denotes a dialyzer, reference sign 407 denotes a venous pressure monitor, reference sign 408 denotes an air trap and air detector, reference sign 409 denotes an air detector clamp, and reference sign 410 denotes clean blood returned to the body.

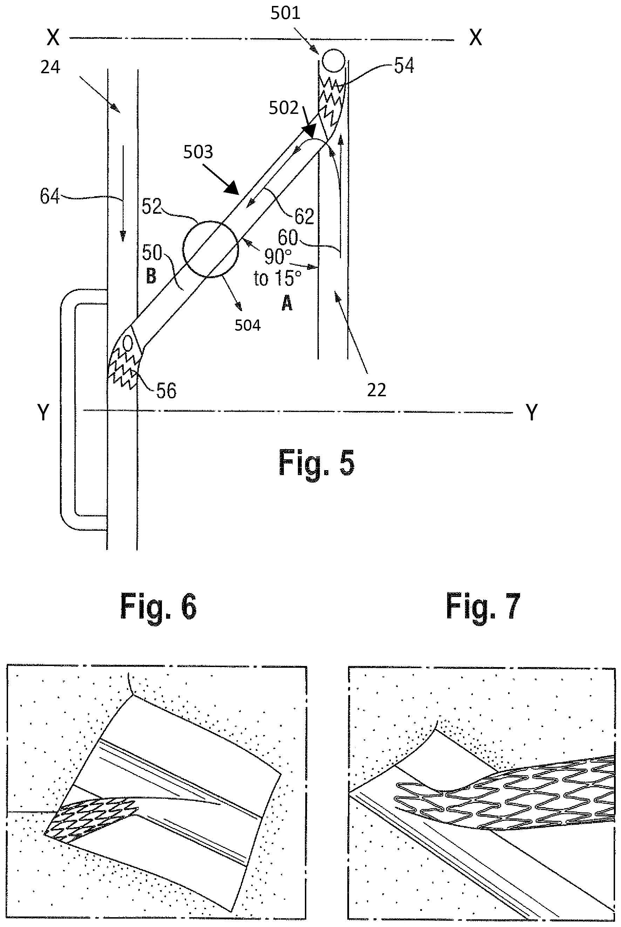

Turning to Fig. 5, reference sign 501 denotes an artery access, reference sign 502 denotes percutaneous tunneling access points if necessary, reference sign 503 denotes a stent graft in the pretunneled arm section, and reference sign 504 denotes a needle landing zone with gel pliable around the cylindrical stent graft surface. Moreover, we use again reference 22 to denote the arterial lumen (artery) and 24 to indicate the venous lumen (vein) which are connected by the shunt. Chain dashed line X-X signifies wrist level and chain dashed line Y-Y signifies elbow joint level.

In the present invention, the shunt is in the nature of a stent graft, depicted in Fig. 5 as element 50. It provides a puncture site 52 (otherwise called "needle landing zone") which can be in one embodiment anywhere along the length of the stent graft between the arterial 22 and venous 24 lumens, or in another embodiment a special zone within that length. In any event, the stent graft exhibits, all the way from one end zone 54 to the other end zone 56, a matrix of stenting struts, as such known to those skilled in the art and to skilled readers. At least in that portion of the length of the stent graft 50 that is not within the arterial lumen 22, nor in the venous lumen 24, the stent matrix is covered, to render it fluid-tight, and the covering in the presently preferred embodiment is of expanded polytetrafluoroethylene.

Important to note in Fig. 5 is the orientation of the shunt with respect to the flow directions of blood in the arterial and venous lumens.

In Fig. 5, arrow 60 shows the flow direction of blood in the arterial lumen 22 and it can be seen that the stent graft shunt has a length direction, arrow 62, that is at an acute angle of between 15° and 90° to the upstream, counter-flow, direction in the artery 22. In consequence, arterial blood flowing along the artery 22 is called upon to turn through an obtuse angle of more than 90° in order to flow along the lumen of the stent graft 50.

By contrast, flow 62 in the stent graft merges with downstream flow 64 in the vein 24 at an acute angle B of less than 90°, more or less complementary to the acute angle A between the stent graft and the upstream direction in the artery. In order to facilitate flow from the artery into the shunt, the covering on the stent graft is cropped back from the arterial end 54 of the stent mesh, along a line that takes a slanting angle with respect to the longitudinal axis 62 of the stent graft, so that the slanting line where the covering ends coincides more or less with the luminal wall of the artery 22. The uncovered stent mesh 54 inside the arterial lumen is hardly any impediment to the flow of blood onward down the arterial lumen, but does serve as a useful anchor for the stent graft shunt within the arterial lumen.

Likewise, at the venous end 56 of the shunt, again, the stent graft covering can be cut back to a line that more or less corresponds to the venous wall, and the uncovered end portion 56 of the stent mesh within the venous lumen again acts as a useful anchor without substantially impeding venous flow onward down the lumen 24.

Furthermore, the procedural steps associated with Fig. 5 are as follows:
- Angiographic or ultrasonic visualization of the artery and the vein.
- Defining the tunneling puncture site
- Option 1: Percutaneous tunneling cross over 10°-15° with 1 access point
- Vascular access with Seldinger technique at artery side
- Delivery system: in to out puncture
- Tracking through the tunnel to the venous site
- Delivery system tip punctures and penetrates through arterial wall
- Deployment of stent graft in 2cm overlap to vein and Artery
- Injection of gel subcutaneously at the needle landing zone
- Direct puncture through pliable gel without waiting for graft material maturing in the needle landing zone

The photographs of Figs. 6 and 7 are of artificial lumens made of polymer, but receiving the opposed ends of a stent graft of the general form contemplated for the present invention. It can be seen that the luminal wall of synthetic polymer is resilient enough to squeeze the diameter of the stent graft, so that the diameter of the lumen of the stent graft is locally constricted, where the stent graft passes through the wall of the arterial and venous lumens. However, that restriction is not sufficient to close entirely the lumen of the shunt. Important for the reader to appreciate is that, in this respect, the artificial polymeric lumen is a poor imitation of bodily tissue, which has more capability to accommodate the prosthesis. Over time, the self-expanding stent will expand radially, as the lumen wall tissue (vein and artery} relaxes and undergoes strain, so that there will be little or no local constriction in the shunt flow path through the arterial or venous wall. It is contemplated to use a percutaneous transluminal angioplasty step (familiar as such to skilled readers) to assist in removing the local constriction to the extent judged desirable or necessary.

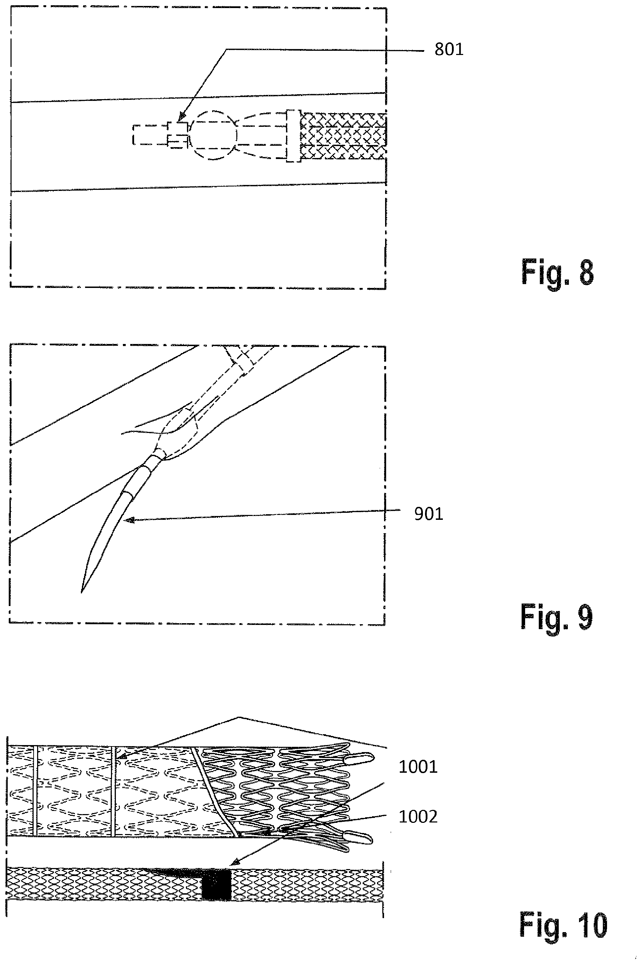

Moving on, to consider Fig. 8, a transparent polymer tube provides a model of an arterial lumen. The photograph in Fig. 8 reveals the details of the distal end of the catheter that is used to deliver the arteriovenous shunt. The open distal end is provided with a radiopaque ring (conveniently of platinum) and immediately proximal of the radiopaque marker ring is an olive-shaped obturator that itself sits immediately distal of a tapered distal end portion of a sheath that confines a radially expansible self-expanding stent graft radially inside the sheath. The sheath is itself provided with a radiopaque marker, again, conveniently, a band of platinum. There is a substantial lumen within the inner tube that carries the obturator. The lumen can accommodate a penetrating means and that, in turn, can define a guidewire lumen. In Fig. 8, reference sign 801 denotes an inner catheter with radiopaque ring followed by an opturator distally from the inwardly tapered catheter.

Moving on to Fig. 9, here with, see the same olive-shaped obturator but this has been advanced distally, on the inner tube, so that there is significant axial spacing between it and the radiopaque marker ring on the stent-confining sheath. Furthermore, distal of the obturator, a penetrating point on a nickel titanium shape memory alloy shaft, has been extended distally, from inside the lumen of the inner tube, from a sheathed non-penetrative disposition, into a distally extended, unsheathed, penetrative disposition, in which it has indeed penetrated the wall of the synthetic artery, and has been further distally advanced until the olive-shaped obturator is located within the puncture of the arterial wall made by the penetrating point. In Fig. 9, reference sign 901 denotes a centergrinded NITINOL stylet with shape set to get a bended configuration of the delivery system pinpointing to the vessel wall.

Not shown in Fig. 9 is a pre-prepared tunnelled channel from the puncture point of the arterial lumen to an intended puncture point of a venous lumen, along which the NITINOL stylet now advances, until the penetrating point punctures the wall of the venous lumen. It is contemplated that the delivery catheter inner tube that terminates in the penetrating point defines a guidewire lumen and that the guidewire is advanced past the penetrating point into the venous lumen. Then, even after proximal withdrawal of the penetrating point into a sheathed non-penetrating disposition, the delivery catheter can be advanced along the guidewire until the obturator olive has passed through the puncture in the wall of the venous lumen and enters the lumen.

At this point, the way is clear for the delivery catheter to be advanced, far enough to advance the leading end of the stent graft, still within the confining sheath of the catheter, into the venous lumen. With percutaneous slits, previously used to create the tunnelled channel for the shunt catheter, progress of the stent graft within its confining sheath can be observed directly visually, and coloured or other visually recognisable markers on the stent graft can be viewed through the slits to ensure that the desired length of the respective end portions of the stent graft are safely located inside the lumen of the artery and vein respectively. When the stent graft is exactly in position, the sheath radially confining the stent graft can be progressively withdrawn proximally analogously to the well-known way of deploying a stent graft or bare stent, until the sheath is fully proximal of the proximal end of the stent in the arterial lumen. After that, the inner shaft of the delivery catheter, complete with obturator olive and marker band and penetrating point, can all be withdrawn through the lumen of the expanded stent graft.

Readers will grasp that the proximal end of the catheter will have a hand-held actuator (as is conventional for delivery of stent grafts trans-luminally). However, the hand unit for the present invention must be capable of rotating the catheter about its long axis to an extent such that the penetrating point can point in the right direction, and the shunt can be given the rotational orientation that will line up the slanting line at the end of the stent covering with the wall of the arterial and, respectively, venous lumens at each end of the shunt.

Once the stent graft is in position, a special gel can be injected through the skin, or introduced through one of the percutaneous slits, to sit as a pad or cushion between the stent graft needle landing zone and the skin layers of the patient. The gel cushions the stent graft and helps to seal it after successive penetrations by the needles of a dialysis machine.

As noted in the text on Fig. 5, the system described above has the potential to permit dialysis with a dialysis machine, more or less immediately after installation of the shunt, without having to wait, as conventionally, for the graft material to mature.

In Fig. 10, reference sign 1001 denotes colored marking on the sheath and on the stent graft for visualized orientation during deployment, and reference sign 1002 shows that also platinum rings are located in the angled cut section of the graft material for alternative fluoroscopy visualization.

Bio-active agents can be added to the prosthesis (e.g., either by a coating or via a carrier medium such as resorbable polymers) for delivery to the host's vessel or duct. The bio-active agents may also be used to coat the entire stent. A material forming the stent or coupled to the stent may include one or more (a) non-genetic therapeutic agents, (b) genetic materials, (c) cells and combinations thereof with (d) other polymeric materials.
(a) Non-genetic therapeutic agents include anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anti-coagulants, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms.
(b) Genetic materials include anti-sense DNA and RNA, DNA coding for, anti-sense RNA, tRNA or rRNA to replace defective or deficient endogenous molecules, angiogenic factors including growth factors such as acidic and basic fibroblast growth factors, vascular endothelial growth factor epidermal growth factor, transforming growth factor alpha and beta, platelet-derived endothelial growth factor, platelet-derived growth factor, tumor necrosis factor alpha, hepatocyte growth factor and insulin like growth factor, cell cycle inhibitors including CD inhibitors, thymidine kinase ("TK") and other agents useful for interfering with cell proliferation the family of bone morphogenic proteins ("BMP's"),BlVfiP-2, BMP-3, BMP-4, BMP-5, BMP-6 (Vgr-1), BMP-7 (OP-1), BMP-8, BMP-9, BMP-10, BMP-1, BMP-12, BMP-13, BMP-14, BMP-15, and BMP-16. Desirable BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA"s encoding them.
(c) Cells can be of human origin (autologous or allogeneic) or from an animal source (xenogeneic), genetically engineered if desired to deliver proteins of interest at the deployment site. The cells may be provided in a delivery media. The delivery media may be formulated as needed to maintain cell function and viability.
(d) Suitable polymer materials as a coating or the base material may include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (for example, BAYHDROL® fibrin, collagen and derivatives thereof, polysaccharides such as celluloses, starches, dextrans, alginates and derivatives, hyaluronic acid, squalene emulsions. Polyacrylic acid, available as HYDROPLUS® (Boston Scientific Corporation, Natick, Mass.), and described in U.S. Pat. No. 5,091,205, the disclosure of which is hereby incorporated herein by reference, is particularly desirable. Even more desirable is a copolymer of polylactic acid and polycaprolactone.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above.

## Claims

1. A system for facilitating dialysis by creating an arteriovenous fistula with a shunt, the shunt having a dialysis needle landing zone, the system comprising:
a catheter for advancing a covered stent carried near the distal end of the catheter along a bodily lumen into a position in which the stent can function as an arteriovenous shunt between a first and a second bodily lumen, said catheter including means to broach radially outwardly a wall of said first lumen and radially inwardly a wall of said second lumen, and then deploy the stent,
a covered radially-expansible stent (50), capable of being advanced by the catheter along a bodily lumen, and capable of performing as an arteriovenous shunt, and with a covering that stops short of one end of the stent; **characterized in that**:
said covering stops short of the stent end at a line that slants to the longitudinal axis of the stent at an angle intermediate between 0° and 90°,
and further **characterized by**:
a means for injection of a pillow or pad of gel to be located subcutaneously between a puncture site (52) of the shunt and overlying cutaneous layers of natural tissue for sealing the puncture site after its puncture in its needle landing zone by a dialysis needle and after withdrawal of the needle.

2. System as claimed in claim 1, wherein the broaching means is a penetrating point at the distal end of the catheter.

3. System as claimed in claim 2, including means to move the point between a distally advanced unsheathed penetrative disposition and a proximally retracted sheathed non-penetrative disposition.

4. System as claimed in claim 2 or 3, wherein the point is capable of facing at an angle to the longitudinal axis of the distal end zone of the catheter, for broaching said wall of said first lumen.

5. System as claimed in any one of claims 1 to 4 and including an obturator at the distal end of the catheter, distal of the location near the distal end where the stent is carried.

6. System as claimed in claim 5, wherein the obturator has an olive shape.

7. System according to any one of the preceding claims, wherein the covering of the stent comprises ePTFE.

8. System according to any one of the preceding claims, wherein the stent is equipped with at least one radiopaque marker.

9. System according to any one of the preceding claims, wherein the stent is equipped with at least one visual marker, that is adapted to be viewed directly by the unaided human eye.

10. System according to claim 9, wherein the visual marker has a colour different from adjacent areas of the stent.

## Patentansprüche

1. System zum Erleichtern von Dialyse durch Erzeugen einer arteriovenösen Fistel mit einem Shunt, wobei der Shunt eine Dialysenadelanlegezone aufweist, wobei das System umfasst:
einen Katheter zum Vorschieben eines in die Nähe des distalen Endes des Katheters beförderten bedeckten Stents entlang eines Körperlumens in eine Position, in der der Stent zwischen einem ersten und einem zweiten Körperlumen als ein arteriovenöser Shunt fungieren kann, wobei der Katheter Mittel beinhaltet, um eine Wand des ersten Lumens radial nach außen und eine Wand des zweiten Lumens radial nach innen anzustechen, und anschließend den Stent einzusetzen,
einen bedeckten radial-expandierbaren Stent (50), der in der Lage ist, durch den Katheter entlang eines Körperlumens vorgeschoben zu werden, und in der Lage ist, als ein arteriovenöser Shunt zu wirken, und mit einer Abdeckung, die kurz vor einem Ende des Stents aufhört; und **dadurch gekennzeichnet, dass**:
die Abdeckung kurz vor dem Ende des Stents an einer Linie aufhört, die sich zu der Längsachse des Stents bei einem Winkel, zwischenliegenden zwischen 0 ° und 90 °, neigt,
und weiter **gekennzeichnet durch**:
ein Mittel zum Einspritzen eines Gelkissens oder -pads, das zum Abdichten der Punktionsstelle nach ihrer Punktion in ihrer Nadelanlegezone durch eine Dialysenadel und nach Zurückziehen der Nadel subkutan zwischen einer Punktionsstelle (52) des Shunts und darüber liegenden kutanen Schichten von natürlichem Gewebe lokalisiert werden soll.

2. System nach Anspruch 1, wobei das Anstechmittel eine Durchdringungsspitze an dem distalen Ende des Katheters ist.

3. System nach Anspruch 2, beinhaltend Mittel, um die Spitze zwischen einer distal vorgeschobenen umhüllungsfreien Durchdringungsdisposition und einer proximal zurückgezogenen umhüllten Nichtdurchdringungsdisposition zu bewegen.

4. System nach Anspruch 2 oder 3, wobei die Spitze in der Lage ist, der Längsachse der distalen Endzone des Katheters zum Anstechen der Wand des ersten Lumens unter einem Winkel zugewandt zu sein.

5. System nach einem der Ansprüche 1 bis 4 und beinhaltend einen Obturator an dem distalen Ende des Katheters, distal von der Stelle in der Nähe des distalen Endes, wohin der Stent befördert wird.

6. System nach Anspruch 5, wobei der Obturator eine Olivenform aufweist.

7. System nach einem der vorstehenden Ansprüche, wobei die Abdeckung des Stents ePTFE umfasst.

8. System nach einem der vorstehenden Ansprüche, wobei der Stent mit mindestens einem strahlungsundurchlässigen Marker ausgestattet ist.

9. System nach einem der vorstehenden Ansprüche, wobei der Stent mit mindestens einer visuellen Markierung ausgestattet ist, die dazu ausgelegt ist, direkt durch das menschliche Auge ohne Hilfsmittel betrachtet zu werden.

10. System nach Anspruch 9, wobei die visuelle Markierung eine Farbe aufweist, die sich von benachbarten Bereichen des Stents unterscheidet.

## Revendications

1. Système destiné à faciliter une dialyse en créant une fistule artérioveineuse avec un shunt, le shunt ayant une zone de réception d'aiguille de dialyse, le système comprenant :
un cathéter pour faire progresser une endoprothèse enveloppée portée près de l'extrémité distale du cathéter le long d'une lumière corporelle dans une position dans laquelle l'endoprothèse peut fonctionner comme un shunt artérioveineux entre une première et une seconde lumière corporelle, ledit cathéter comprenant un moyen pour aborder radialement vers l'extérieur une paroi de ladite première lumière et radialement vers l'intérieur une paroi de ladite seconde lumière, puis déployer l'endoprothèse,
une endoprothèse enveloppée d'expansion radiale (50), susceptible de progresser par le cathéter le long d'une lumière corporelle, et susceptible de faire office de shunt artérioveineux, et doté d'une enveloppe qui s'arrête près d'une extrémité de l'endoprothèse ; et **caractérisé en ce que** :
ladite enveloppe s'arrête près de l'extrémité de l'endoprothèse au niveau d'une ligne qui s'incline vers l'axe longitudinal de l'endoprothèse suivant un angle compris entre 0 et 90°,
et **caractérisé en outre par** :
un moyen pour l'injection d'un coussin ou d'un coussinet de gel à placer par voie sous-cutanée entre un site de ponction (52) du shunt et recouvrant des couches cutanées de tissu naturel pour obturer le site de ponction après sa ponction dans sa zone de réception d'aiguille par une aiguille de dialyse et après le retrait de l'aiguille.

2. Système selon la revendication 1, dans lequel le moyen de brochage est un point de pénétration au niveau de l'extrémité distale du cathéter.

3. Système selon la revendication 2, incluant un moyen pour déplacer le point entre une disposition pénétrante non gainée progressant distalement et une disposition non pénétrante gainée rétractée proximalement.

4. Système selon la revendication 2 ou 3, dans lequel le point est susceptible de faire face suivant un angle par rapport à l'axe longitudinal de la zone d'extrémité distale du cathéter, pour aborder ladite paroi de ladite première lumière.

5. Système selon l'une quelconque des revendications 1 à 4, et incluant un obturateur au niveau de l'extrémité distale du cathéter, distale de l'emplacement à proximité de l'extrémité distale où l'endoprothèse est amenée.

6. Système selon la revendication 5, dans lequel l'obturateur a une forme d'olive.

7. Système selon l'une quelconque des revendications précédentes, dans lequel l'enveloppe de l'endoprothèse comprend du ePTFE.

8. Système selon l'une quelconque des revendications précédentes, dans lequel l'endoprothèse est équipée d'au moins un marqueur radio-opaque.

9. Système selon l'une quelconque des revendications précédentes, dans lequel l'endoprothèse est équipée d'au moins un marqueur visuel, qui est conçu pour être vu directement par l'œil humain sans aide.

10. Système selon la revendication 9, dans lequel le marqueur visuel a une couleur différente des zones adjacentes de l'endoprothèse.
